# EUROPEAN PATENT SPECIFICATION

(11) **EP 2 215 470 B1**
(45) Date of publication and mention of the grant of the patent: **30.04.2014**
(21) Application number: 07816226.0
(22) Date of filing: 02.11.2007
(51) Int. Cl.: G01N 33/49

(54) **METHOD AND APPARATUS FOR MONITORING SPATIAL FIBRIN CLOT FORMATION**
VERFAHREN UND VORRICHTUNG ZUR ÜBERWACHUNG RÄUMLICHER FIBRINGERINNSELBILDUNG
PROCÉDÉ ET APPAREIL POUR SURVEILLER LA FORMATION D'UN CAILLOT DE FIBRINE DANS L'ESPACE

(43) Date of publication of application: 11.08.2010
(73) Proprietor: Medplast S.A., 1950 Sion (CH); Medical Innovations Ltd., Moscow 127254 (RU)
(72) Inventor: ATAULLAKHANOV, Fazoil, Moscow 117419 (RU); SARBASH, Vasilii, Moscow 105066 (RU); OVANESOV, Mikhail, Moscow 103460 (RU); PANTELEEV, Mikhail, Mytishchi 141004 (RU)
(74) Representative: Rüedi, Regula Béatrice
(86) International application number: PCT/CH2007/000543
(87) International publication number: WO 2009/055940

(56) References cited:
- EP-A- 0 637 744
- US-A- 5 504 011
- FAXÄLV LARS ET AL: "Imaging of blood plasma coagulation and its propagation at surfaces." JOURNAL OF BIOMEDICAL MATERIALS RESEARCH. PART A 15 JUN 2008, vol. 85, no. 4, 28 September 2007 (2007-09-28), pages 1129-1134, XP007905639 ISSN: 1552-4965 cited in the application

## Description

### Technical Field

The present invention.relates to a method and an apparatus for the monitoring of spatial fibrin clot formation in multiple samples.

### Background Art

The problem of modelling blood coagulation in vitro for diagnostical and basic research purposes is long-standing. A number of methods and devices for measuring clotting parameters of plasma have been proposed; however, the vast majority of these methods (e.g. activated partial thromboplastin time test, thrombelastography, thrombin generation assay) monitor clotting times or other homogenous parameters. In other words, in these tests where the sample of blood (plasma) is uniformly mixed with the activator, coagulation of the sample as a whole is monitored. This is the simplest approach, but it is non-physiological, because blood coagulation in vivo is a spatially non-uniform phenomenon. Diffusion of reactants plays a critically important role in this process.

There are few methods which take into consideration the role of spatial heterogeneity and diffusion. Among the most relevant methods which take these parameters into account is the cell-based coagulation assay (Monroe et al., Br J Haematol. 1994; 88(2): 364-371 followed by a series of studies). This method is based on the use of a conventional spectrofluorimeter. It involves monitoring thrombin generation by subsampling with the help of a fluorogenic substrate in a reconstituted system of plasma proteins and platelets, where coagulation is activated by a monolayer of tissue factor-expressing cells (monocytes). Although this is a sensitive method, its major. disadvantage is that it is not truly spatial: although reactants are distributed heterogeneously, the observed signal is an integral characteristic of the coagulation process. Mixing by the pipette during sample acquisition also makes spatial heterogeneity less pronounced. The method is immensely labor-intensive and time-consuming. It has been used for basic research and pharmacological studies only. In the present form it is impossible to use for diagnostics, because the method cannot be used for plasma clotting without modifications since formation of fibrin clot will prevent possibility of sampling.

A second approach is the spatial clotting method (Ataullakhanov et al. Biochim Biophys Acta. 1998; 1425(3): 453-468; Sinauridze et al. Biochim Biophys Acta. 1998; 1425(3): 607-616; Ovanesov et al. Biochim Biophys Acta. 2002; 1572(1): 45-57; Ovanesov et al. Thromb Haemost. 2003; 89(2): 235-242; Ovanesov et al. J Thromb Haemost. 2005; 3(2): 321-331; Panteleev et al. Biophys J. 2006; 90(5): 1489-1500 ). This method allows studies of spatial clot formation by video-microscopy monitoring of thrombus formation in recalcified plasma induced by bringing plasma in contact with an activator, either glass or fibroblast monolayer. This method is well-established as sensitive and reproducible approach. It has been actively used for the studies of blood coagulation regulation. In addition to basic research, spatial coagulation abnormalities in various abnormal states were studied (hemophilias A, B, and C; deficiencies of coagulation factors X, VII, II and fibrinogen; sepsis and massive trauma; plasma dilution during transfusion therapy, etc.) and mechanisms of action of therapeutic agents were investigated (factor VIII concentrates, recombinant activated factor VII (Novoseven®, Novo Nordisc, Denmark), antithrombin III concentrates, etc.). Despite the advantages, the method and the apparatus for spatial clotting used previously are inadequate for practical use. The method does not allow simultaneous measurement of more than one sample, which is not convenient for basic research and completely prevents wide clinical use. The required minimal quantity of plasma is 300 µl, which several-fold exceeds the quantity used in standard clotting tests (100 µl). Activation is performed by either fibroblast monolayer or glass; the former method is poorly reproducible, expensive and time-, labor-, and material-consuming (cell culturing facility is needed), the latter method is non-physiological (activation by glass is via the intrinsic pathway, which does not function in vivo). To keep the sample at 37°C, the cuvette is thermostabilized by keeping it in a water incubator with a stirring device; however, this results bubble formation impairing the signal quality.

A third approach involves the imaging of blood plasma coagulation and its propagation from surfaces (see Faxalv et al. J Biomed Mater Res A. 2007; epublished ahead of print. DOI: 10.1002/jbm.a.3152) published as Faxàlv et al. (2008), J Biomed Mater Res, Vol 85(4), pp. 1129-1134 . This approach is essentially similar to the spatial clotting method above and relies on the time-lapse image capture of light scattering from the fibrin network developing in a cuvette. Activation was done by glass only. The major difference was the use of standard spectrophotometric cuvettes instead of special cuvettes used in the spatial clotting method. Several cuvettes can by monitored simultaneously, but with low resolution and without automatization. Although the method allows for the use of standard spectrophotometric cuvettes and ability to monitor several samples simultaneously, its major disadvantage is that the method requires very large quantities of plasma (1500 µl). Furthermore, it provides a low spatial resolution for experiments with several samples (100 µm instead of 10 µm in "Spatial clotting method"), and is performed by non-physiological activation (by glass only). There is no automatization, and the method is time-consuming and labor-intensive. The method was designed for material testing only, and its suitability for diagnostic purposes, basic and pharmacological research is limited.

A forth approach consists in the spatiotemporal clotting on patterned supported phospholipid bilayers with reconstituted human tissue factor (Kastrup et al. Proc Natl Acad Sci U S A. 2006; 103(43): 15747-17752). In this method, a microfluidic chamber was used to contain freshly recalcified plasma over the patterned lipid surface. Bright-field microscopy was used to detect formation of fibrin. In this method, the optical axis is perpendicular to the activator surface, which does not allow monitoring spatial propagation in this direction. Only clot formation and its propagation along the activator can be observed. The principal advantage of this method is its ability to observe clotting on the patterned activating surface and study the mechanisms of clotting initiation. However, this is a narrow and specific problem. Suitability of the method for other types of basic research, for diagnostic purposes and pharmacological research is limited.

A similar approach intended not for fibrin clot formation but for agglutination pattern analysis is described EP 0 637 744. In this method for analyzing the agglutination pattern of blood, the sample is filled into a cassette having a multitude of wells, each well comprising transparent glass microbeads. The blood samples are tested for the presence of agents capable of inducing agglutination. The number, size and distribution of the agglutinates is an indication of the strength of the reaction. An illuminated image of the solution is produced on an array of pixels and to each pixel in the illuminated image, a data value is assigned, representing the intensity of the illuminated image on the pixel. According to a predetermined program, these data values are processed to determine if an agglutination pattern is present and, if so, to classify that pattern.

Thus, there is a need in the art for an improved method and in particular an apparatus which overcomes one or more of the above mentioned drawbacks.

### Disclosure of the Invention

Hence, it is a general object of the invention to provide a high resolution method and in particular an apparatus which allows for a sensitive, reproducible in vitro modelling of blood coagulation (specifically, the processes of fibrin clot formation and/or dissolution), taking into account the role of spatial heterogeneity and diffusion, and which are apt for the simultaneous monitoring of multiple samples, particularly important in clinical monitoring.

The present innovation discloses inter alia a cuvette assembly 26, a cuvette holder 13, an apparatus 14 comprising the cuvette holder and an optical detection device, as well as a method, which constitute a further development of the spatial clotting method, much simpler to manufacture and to use, providing more information, allowing automatization, and being apt for the use in a clinical environment.

In a first aspect, the present invention provides a cuvette assembly 26 for photometric analysis, comprising a cuvette, an insert and an activator of coagulation, wherein the cuvette 1 is segmented into a multiplicity of wells 2 by segmentation walls 4; the insert 5 comprises a multiplicity of projections 6 protruding from an area 7, which are adapted to fit into the wells 2 of said cuvette 1; and the activator is selected from the class of physiological and non-phsiological activators of coagulation and wherein said activator is located on the insert projections 6, preferably at the bottom edge 24 of the projections 6.

Preferably, the wells 2 of the cuvette 1 are plan-parallel and/or of rectangular shape in a cross-sectional view.

More preferably, the wells 2 of the cuvette 1 are arranged in one line and have an equitable volume.

In a preferred embodiment, the cuvette 1 is made of plastic light-transmissive material, preferably of polystyrol. However, other biologically inert materials may be used, such as polyvinylchloride or polymethyl methacrylate.

In a further preferred embodiment, the number of projections 6 of the insert 5 corresponds to the number of wells 2 in the cuvette 1.

In another preferred embodiment, the insert 5 when fitted into the cuvette 1 reduces the effective distance between the surface of the insert 5 and the inner surface of the cuvette 1 to 0,1 mm or less, preferably to 0,05 mm.

In still another preferred embodiment, the insert 5 carries on each projection 6 the same or a different activator, or wherein not all projections 6 carry an activator.

In still another preferred embodiment, the projections 6 of insert 5 are replaceable so that a desired combination of activators for a specific experiment can be obtained by setting the projection 6 in a required composition into the insert 5. Thus, the invention also relates to a cuvette assembly wherein the projections 6 of the insert 5 are detachably or non-detachably connected to the area 7.

In an advantageous embodiment, the insert 5 is made of plastic, preferably of polystyrol, polyvinylchloride or polymethyl methacrylate. The invention also encompasses the use of other materials for the insert 5, such as other biologically inert materials.

In another advantageous embodiment, the activator is a physiological coagulation activator, e.g. tissue factor, thrombin, a layer of tissue-factor-expressing cells, tissue samples, etc. For experiments involving monitoring of not only clot formation, but also of clot dissolution, fibrinolysis activator (tissue plasminogen activator (tPA), urokinase plasminogen activator (uPA), etc.) can be used. For studies of biomaterial compatibility, non-physiological activators, e.g. glass, samples of implantable devices/materials and artificial organs/materials, etc. can be used. The activators, preferably proteins, are immobilized on the surface by known chemical procedures, e.g. chemical bonding.

The cuvette assembly 26 disclosed herein is preferably used for monitoring spatial fibrin clot formation *in vitro.*

Further disclosed, but not part of the invention, is a cuvette holder. As used herein, the term "cuvette holder" relates to a device which fixes a cuvette so that chemical or biochemical reactions occurring within the cuvette can be monitored by appropriate equipment.

The cuvette holder described herein comprises a thermostat 10 to thermostabilize the cuvette 1 and means for holding the cuvette 1 within the thermostat 10 and along an optical path. As used herein, the term "thermostat" refers to a chamber with a temperature controlled fluid 28. The thermostat 10 is filled with a fluid 28; suitable fluids 28 include gases, such as N₂ and air; liquids, such as aqueous, alcohols or polyols, most preferably water. Furthermore, the thermostat 10 including the fluid 28 is at least partially light-transmissive so that chemical or biochemical reactions within the cuvette assembly 26 can be registered via an optical device. The fluid 28 is also within the optical path. Preferably, the cuvette holder 13 is thermostabilized and thermoisolated.

In a preferred embodiment, the cuvette holder is adapted in that the cuvette 1 is arranged perpendicular to the optical path and in an inclined manner with regard to the vertical line, preferably by 10-40°. However, the cuvette plane should be strictly perpendicular to the optical path.

In a preferred embodiment, the cuvette holder is especially adapted for the cuvette described above.

Also described, but not part of the invention is an apparatus 14 for monitoring spatial fibrin clot formation and/or dissolution in vitro comprising the cuvette holder described herein and an optical detection device.

The above disclosed apparatus can comprise a cuvette holder 13 and an optical detection device wherein the cuvette holder 13 arranges a cuvette 1 perpendicular to the optical path and in an inclined manner with regard to the vertical line, preferably by 10-40°. The cuvette holder is preferably the cuvette holder 13 disclosed herein. Typically, the cuvette plane is strictly perpendicular to the optical path.

In another aspect, the invention describes a method for *in* vitro monitoring of spatial fibrin clot formation and/or dissolution, comprising the steps of:
A) in case of clot formation
   (a) Providing a cuvette containing one or more samples of blood plasma;
   (b) Contacting plasma with a physiological activator of coagulation; and
   (c) Register the growth of the fibrin clot in function of time.
B) in case of clot dissolution
   (a) Providing a cuvette containing one or more samples of blood plasma comprising one or more fibrin clots;
   (b) Contacting plasma with an activator of fibrinolysis; and
   (c) Register the dissolution of the fibrin clot in function of time.

The method is preferably performed at controlled temperature, preferably at physiological temperature, i.e. around or at 37°C.

The cuvettes of step (a) are the above disclosed cuvettes 1. The distribution of samples into the cuvette 1 may occur using a multipipette, which allows for the automatization of the method.

More preferably, the cuvette 1 is placed into the cuvette holder 13 disclosed herein. Most preferably, the method is performed within the apparatus 14 disclosed herein.

Step (b) is performed by inserting the above described insert 5 into the cuvette 1. The comb-like insert 5 carries an activator on the surfaces of the projections 6, preferably at the bottom edge 24 of the projections 6. When the activator present on the edges 24 of the projections 6 comes in contact with the plasma, the fibrin clot begins to form on the activator surface and propagates into the bulk of plasma.

Preferably, the activator is tissue factor; however, other activators, such as thrombin, tissue factor expressing cells or tissue samples may be used. Alternatively, the activator may be non-physiological, e.g. glass or plastic, which is being tested for its biocompatibility. For experiments on blood clot dissolution, activators of fibrinolysis (such as tissue plasminogen activator) can be used.

The term "plasma" as used herein comprises blood plasma, platelet-rich blood plasma, recalcified blood plasma and fractions of plasma. For specific research applications, plasma can be mixed with various reagents (additional activators, inhibitors, pharmaceuticals, etc.). To monitor fibrinolysis, plasma can be mixed with a solution of a fibrinolysis activator (for example, urokinase plasminogen activator or streptokinase). Also, for research purposes, specific reagent mixtures of coagulation proteins, calibration solutions etc. can be used instead of plasma. Thus the term "plasma" is to be understood in this broad sense, also including these modified plasma types.

Fibrin clot growth is illuminated, for example by light emitting diodes. The light scattered by the growing fibrin clot can be registered by an optical detection device 15, e.g. by a CCD camera. The registered data, e.g. captured time-lapse images of growing thrombus, are then analyzed, preferably, digitally by a computer. For example, the main parameters which can be determined by the method disclosed herein are the velocity of clot growth, lagtime and time of coagulation.

Also described, but not part of the invention, is an activator of blood coagulation or fibrinolysis being immobilized on, e.g. chemically bonded to, a surface. This is useful for in vitro monitoring spatial fibrin clot formation or dissolution, respectively. The activator of blood coagulation may be a protein, such as tissue factor or thrombin, or cells or tissues expressing tissue factor. Examples of activators for fibrinolysis include tPA and uPA.

The principal advantage of the herein disclosed method and apparatus 14 for monitoring spatial fibrin clot formation from those of the prior art is that only a small volume of plasma is needed. With as little as 20 µl (instead of 300 up to 1500 µl, i.e. 75-fold less than the only other similar system reported previously, and 5-fold less than the minimal plasma amount required for standard clotting assays), reliable results of high resolution can be produced.

Furthermore, the cuvettes 1 of the present invention are easy to use and require only a minimal set of operations, which do not require any special skills. In contrast, the prior art devices require a professional biochemist with hand skills above average in order to operate.

The apparatus 14 is simple in operation and can be used by assistant staff, in contrast to prior art solutions, which require a skilled biochemist or two. The use of convenient dispensable cuvettes 1 and inserts 5 minimizes the required set of operations to a minimum (insert the cuvette 1, load plasma, insert the activator present on the insert 5).

The invention can be used to monitor not only clot formation, but also the process of fibrin clot lysis ("dissolution" or fibrolysis).

Moreover, the test samples are quickly loaded on the cuvettes 1 described herein. Sample loading requires little time, no more than 1-2 min. The usual experimental time is 25-30 min; images are taken each 10 sec. The normal pattern of thrombus formation comprises a lag time of 2-5 min (during this time after activation, nothing is seen; the lag time strongly depends on the activator type), followed by fibrin clot propagation into the bulk of plasma at a rate of 30-40 µm/min. In pathological conditions, the lag time can be prolonged or shortened; the clot growth rate can be increased or decreased. In addition, spontaneous, activator-independent clotting can be observed in the cuvette 1 in case of procoagulant changes in plasma.

Most importantly, several samples can be monitored simultaneously by using different activators in an automated manner: Using the cuvettes 1 disclosed herein allows for simultaneous loading of plasma into all wells, e.g. by distributing the sample by conventional multipipettes, and use of the insertable activator allows simultaneous activation of coagulation with a single motion. In an embodiment wherein a specially designed disposable plastic cuvette 1 with four wells and a corresponding insert 5, spatial clot formation may thus be monitored in up to four different samples with up to four different activators simultaneously. The samples can be simultaneously loaded (e.g. with a multichannel pipette) and simultaneously activated with the comb-like insert.

The principal advantage of this invention is the ability to detect clotting disorders, which are poorly or not detectable by conventional coagulation assays, such as activated partial thromboplastin time test, prothrombin time test, thrombelastography or thrombin generations assay; for example, procoagulant changes in plasma. Although this advantage might be partially shared by prior art spatiotemporal methods described before, the proposed use is the only one which is convenient for practical use in a clinical setting.

The invention can be used for diagnostics, biomaterial testing, basic and pharmacological research in thrombosis and haemostasis.

### Brief description of the drawings

The invention will be better understood and objects other than those set forth above will become apparent when consideration is given to the following detailed description thereof. Such description makes reference to the annexed drawings, wherein:
Fig. 1a shows a front-view of the cuvette 1 and Fig. 1b a cross-sectional view of the inner space of the cuvette 1.
Fig. 2a shows a front-view of the comb-like insert 5 for the cuvette 1. Fig. 2b provides a side-view of the insert 5 and Fig. 2c a perspective view of the insert 5. Fig. 2d shows a cross-sectional view of the cuvette assembly 26. Fig. 2e shows a side view of Fig. 2d.
Fig. 3 shows the cuvette holder 13 wherein the cuvette 1 is placed. The holder includes a thermostat 10 that can be accessed via the waterproof door 11.
Fig. 4 shows a perspective view of the principal elements of the apparatus 14.
Figs. 5a and 5b show schematically an arrangement of the inclined positioning of a cuvette 1 in an optical path.

### Modes for Carrying Out the Invention

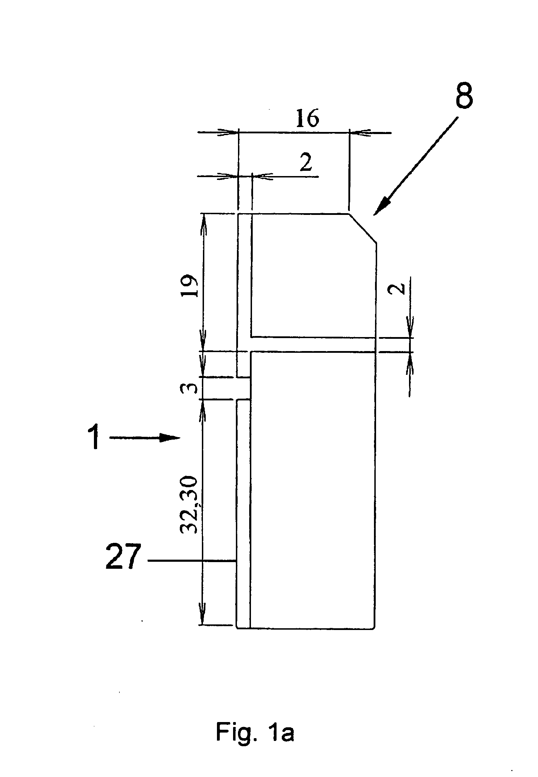

Fig. 1a shows a front-view of the experimental cuvette 1. The cuvette 1 is of substantially cuboid shape.

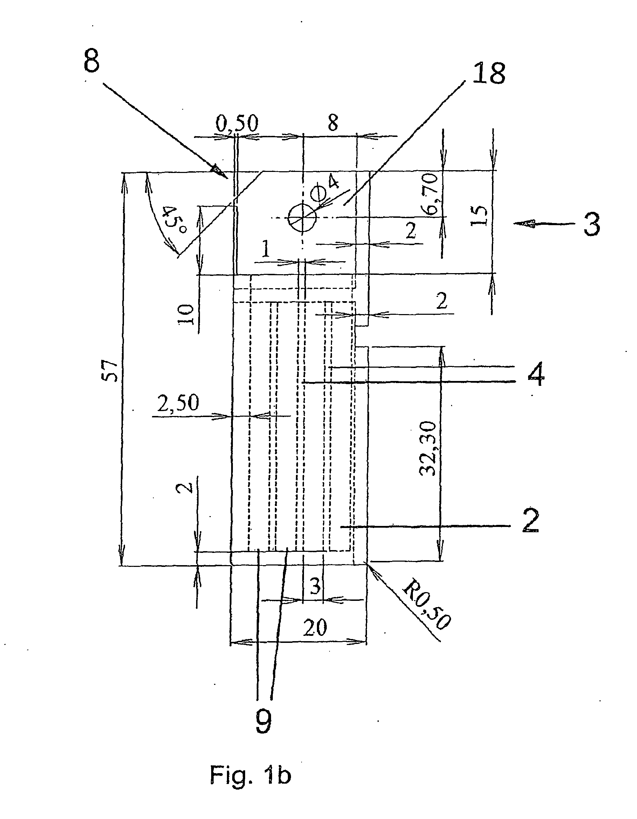

As can be seen in the cross-sectional view of Fig. 1b, the inner space of the cuvette 1 is divided by segmentation walls 4 into four wells 2 of equitable size. Each well 2 has a rectangular section of 3x1 mm². However, the invention is not limited to the indicated dimensions; for example, more wells 2 with smaller rectangular sections are possible. In a preferred embodiment, the overall design of the cuvette 1 and the wells 2 are designed such that only a small sample volume is necessary to provide a reliable result. In the embodiment shown in Figs. 1a and b, a sample volume of only 20 µl is necessary to achieve a reliable result.

Preferably, the segmentation walls 4 segment the cuvette 1 into a number of parallely and serially aligned wells 2 (see Fig. 1b). The width of the chambers or wells 2 is preferably equitable.

In this embodiment, the segmentation walls 4 are not as high as the entire height of the cuvette 1. However, the segmentation walls 4 may have different heights, e.g. be as high as the top of the cuvette.

In the head part 18 of the cuvette 1 is a holding means 19, which allows fixing the insert 5 to the cuvette 1. Here, the holding means 19 is designed as a knub 19; however, other embodiments are also possible.

The head part 18 is in the front-view substantially quadratic with an inclined edge. However, the head part 18 may also be differently shaped. The inclined edge area 8 gives an orientation to the cuvette 1, so that the user may unequivocally locate his samples and/or the insert 5 into the cuvette 1. In another embodiment, the cuvette 1 may have a different orientation means 8 in order to orient the cuvette 1, such as a color mark, a sign or a protruding area.

The edges 27 on the outer surface of the cuvette 1 in Fig. 1a also serve for correct positioning and are optional. While edge 8 helps correct orientation of the insert 5 within the cuvette 1, these orientation means 27 are guides preventing incorrect orientation.

In a further preferred embodiment, the cuvettes are wider than deep, preferably, the width is only 1,1 mm or approximately 1,1 mm.

The cuvette 1 is of polystyrol. However, other light-transmissive plastics are also encompassed by the present invention, such as polymethyl methacrylate or polystyrene, for example.

The segmentation walls 4 are of the same material as the cuvette 1; however, in an alternative embodiment, said segmentation walls 4 may be of a different material. As shown in Fig. 1b, the cuvette has four wells 2. However, any other number of wells 2 which is technically feasible and useful is encompassed by the present invention.

Furthermore, in an alternative embodiment, the cuvette 1 may have means on its outer surface 3 for being stably and removably inserted into a cuvette holder as described herein after.

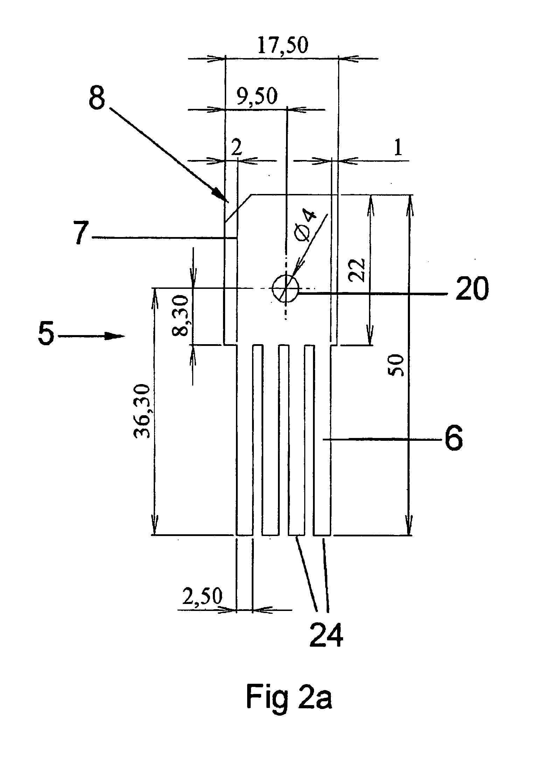

Fig. 2a shows a front-view of the comb-like insert 5 for the cuvette 1. The insert 5 has four projections 6 protruding in a parallel manner from a support 7. The insert 5 of the invention is not limited to four projections, any other number of projections 6 which is technically feasible and useful is encompassed by the present invention. Most preferably, the number of projections 6 corresponds to the number of wells 2 in the cuvette 1.

Furthermore, the projections 6 can be replaceable as to allow easy on-site composing of a needed combination of activators for a specific experimental design.

The area 7 (support) is substantially rectangular, with an inclination 8 on the upper left border, which allows orienting the insert 5 once it shall be inserted into the cuvette 1. By holding the area 7 the insert 5 can be grasped and manipulated. In a further preferred embodiment, the area 7 carries instead of or in addition to the inclined edge other orientation means 8, e.g. a physical mark such as a color mark, a sign or a protruding area. Thereby, the user may easily orient the insert 5 in the wells 2 of the cuvette 1, appointing each of the projections 6 to a specific well 2. In one embodiment, the orientation means 8 may be formed as a guiding area protruding from area 7, which upon insertion guides the insert 5 along the head part 18 of the cuvette 1.

Typically, the insert projections 6 are narrower than the width of the wells 2 of the cuvettes 1, so that the insert 5 may be inserted into the cuvette 1, also in order to allow exit for air. When the insert 5 is placed into the cuvette 1, the remaining space 28 between the surface of the insert 5 and the inner surface of the cuvette 1 is preferably of 0,05 mm, which allows potentially dropped air to escape.

When placed into the cuvette 1 loaded with samples, the projections 6 have direct contact with the samples. However, the length of the projections 6 should be such that they do not reach the bottom 9 of the wells 2. The forming fibrin clot will grow from the bottom edge 24 of the projections 6 towards the bottom of the cuvette 1, i.e. in the remaining space 28 formed between the projections 6 and the bottom 9 of the wells 2.

A circular hole 20 present in the area 7 serves as fixing means 20 which allows connecting the insert 5 with the holding means 19 on the inner surface of the cuvette 1. The fixing means 20 may have a different design. However, holding means 19 and fixing means 20 preferably natch each other.

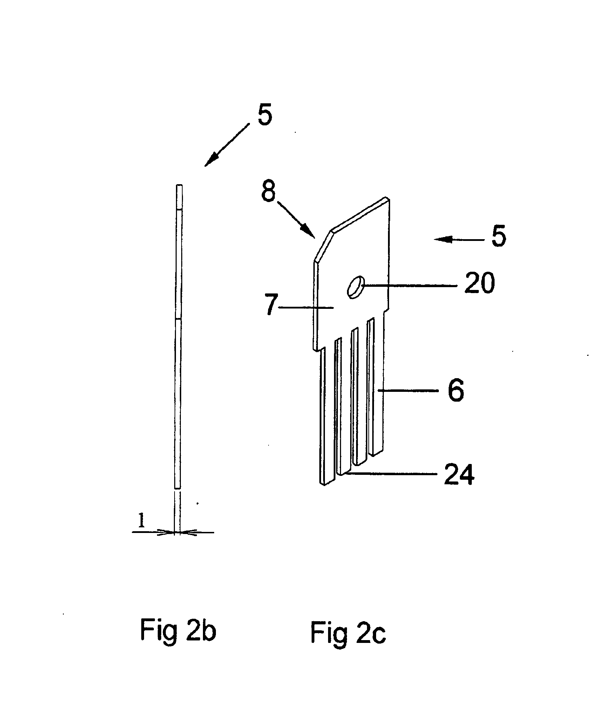

As can be seen in Fig. 2b and Fig. 2c, the insert 5 is more narrow than wide.

The insert 5 is of polystyrol; however, other materials, preferably plastics such as polymethyl methacrylate for example, are also encompassed by the present invention.

The insert 5 carries on its surface, preferably on the bottom edge 24 of the projections 6, an activator of coagulation or fibrinolysis. Various types of coagulation-activating surfaces can be used as activator, but preferably a physiological activator, e.g. an immobilized tissue factor, which allows reproducible physiological initiation of clotting, is used. Alternatively, the insert 5 is made of glass and the presence of a physiological activator may not be necessary. Each projection 6 of the insert 5 may carry the same activator in the same or different concentration, or each projection 6 may carry a different activator. When using the embodiments as shown in Fig. 1b and Figs. 2a-c, spatial clot formation may thus be monitored in up to four different samples with up to four different activators simultaneously.

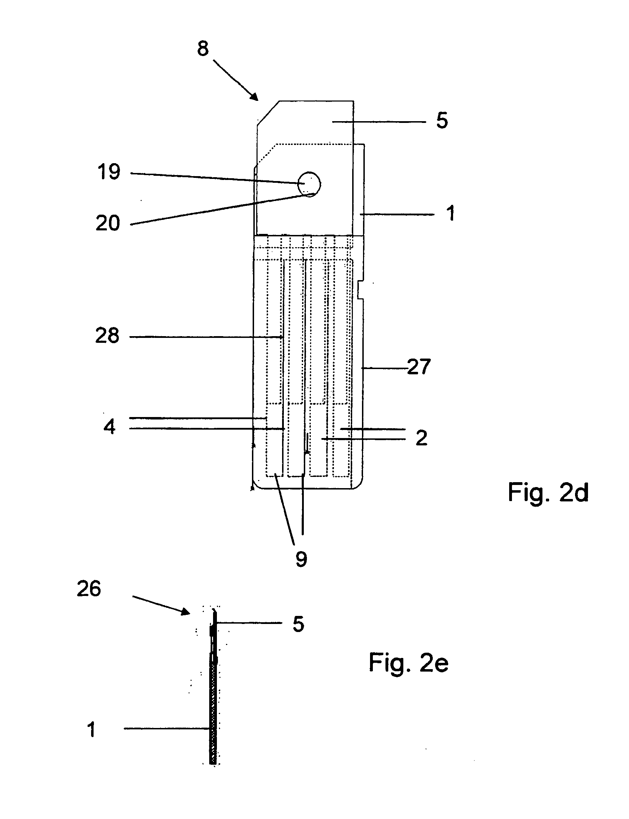

In the cross-sectional view of Fig. 2d, the insert 5 placed into the cuvette 1 is shown. The position of the insert 5 within the cuvette 1 is fixed, since the insert was mounted with its fixing means 20 on the holding means 19 of the cuvette 1. A small space 28 between the projections 6 and the segmentations walls 4 is left, that allows potentially entrapped air to escape. The orientation means 8 of the insert 5 as well as of the cuvette 1 are aligned. As can be seen, the bottom edges 24 of the projections 6 do not reach the bottom of the wells 2. Fig. 2e shows a side view of the insert 5 placed into the cuvette 1.

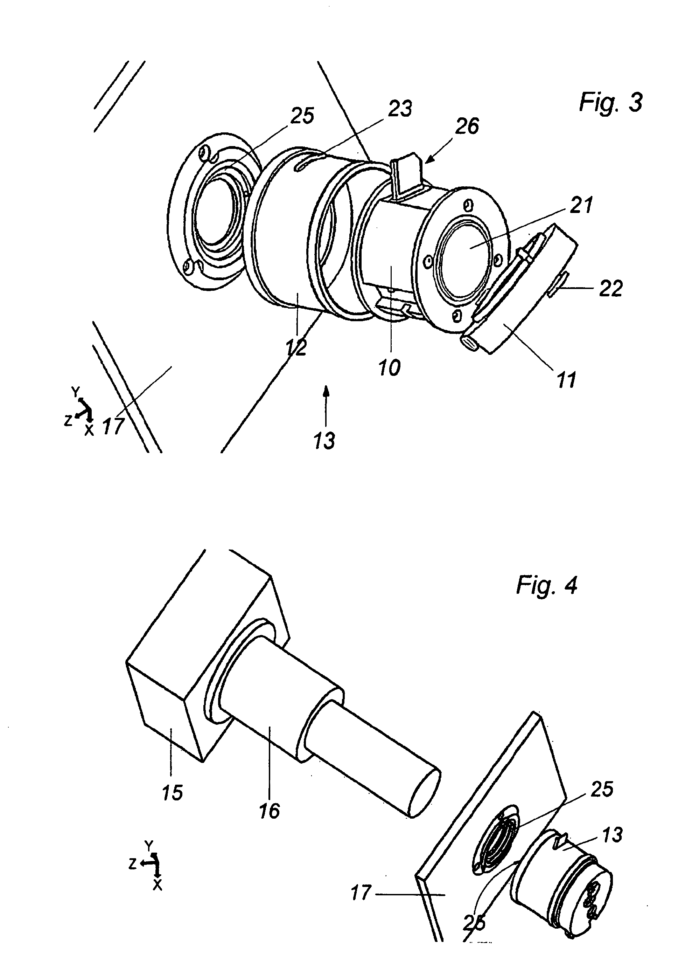

Fig. 3 shows a dismantled cuvette holder 13 which is no subject-matter of the instant invention wherein the cuvette assembly 26 is placed into the thermostat 10.

The thermostat 10 provides thermostabilization and thermoisolation as to minimize possible convection within the different samples located in the cuvette. A fluid 28 is present within the thermostat 10, which is preferably recirculated and filtered and/or comprises an antibacterial agent, in order to ensure a good visibility. However, other embodiments are possible, wherein no thermostabilization and/or thermoisolation occurs.

The thermostat 10 may be accessed via the waterproof door 11 allowing maintenance. Door 11 and thermostat 10 are connected via connecting means 22, such as screws, as shown in Fig. 3.

The thermostat 10 has a visible part 21 allowing imaging and monitoring of clot formation. The thermostat 10 may have one or more visible parts 21 or be entirely transparent.

Fig. 3 further shows a casing 12 with an opening 23 for inserting the cuvette 1, when the thermostat 10 with the door 11 are inserted into the casing 12. Said casing preferably has at least one connecting means 25, such as a winding, which allows connecting the cuvette holder 13 to another item, such as a panel 17.

Fig. 4 shows a perspective view of a
dismantled apparatus 14 which is no subject-matter of the invention, comprising an identification means 15, here a CCD camera with an objective 16, a front panel 17 and the experimental cuvette holder 13 with cuvette 1.

The identification means 15 is an optical detection device, such as a CCD camera, a video camera, photographic film, or current instrumentation such as laser scanning devices, microscopes, or other light detector.

The presence of the front panel 17 is optional. The attachment of the cuvette holder 13 within the apparatus 14 may be solved differently. In case a front panel 17 is used, the cuvette holder 13 can be attached via connecting means 25, such as a winding 25.

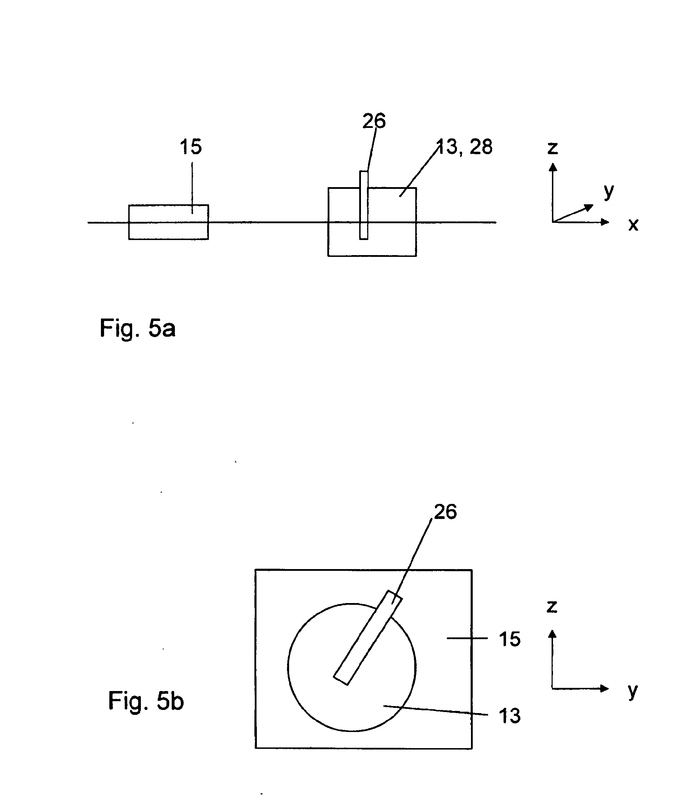

Typically, the cuvette holder 13 is positioned such, e.g. attached to the front panel 17, that the cuvette 1 is placed in a non-vertical position in order to prevent air trapping when the insert 5 is inserted into the cuvette 1. Preferably, the inclination is by 10-40° with reference to the vertical axis (see Figs. 4 and 5).

Optionally (not shown), a casing may protect the apparatus 14.

In Fig. 5a, it is schematically shown that the cuvette 1 or the cuvette assembly 26 are located in the cuvette holder 13. The cuvette holder and the optical detection device are both aligned in the optical path in direction of the x-axis. The perpendicular planes to the optical path is formed in direction of the y- and z-axis. As can be seen, the cuvette 1 or cuvette assembly 26 is arranged perpendicular to the optical path. Fig. 5b is a schematically front view of Fig. 5a and shows that the cuvette 1 or cuvette assembly 26 is not only perpendicular to the optical path, but also inclined with regard to the vertical line.

## Claims

1. A cuvette assembly for photometric analysis, comprising a cuvette segmented into a multiplicity of wells (2) by segmentation walls (4), **characterised by** an insert and an activator, wherein
said insert (5) comprises a multiplicity of projections (6) protruding from an area (7), which are adapted to fit into the wells (2) of said cuvette (1); and
said activator is selected from the class of physiological activators of coagulation, non-physiological activators of coagulation, physiological activators of fibrinolysis or of non-physiological activators of fibrinolysis and wherein said activator is located on the insert projections (6).

2. The cuvette assembly of claim 1, wherein said activator is located at the bottom edge (24) of the projections (6).

3. The cuvette assembly of claim 1 or 2, wherein the wells (2) are plan-parallel and/or of rectangular shape in a cross-sectional view.

4. The cuvette assembly of anyone of the preceding claims, wherein the wells (2) of the cuvette (1) are arranged in one line and have an equitable volume.

5. The cuvette assembly of anyone of the preceding claims, wherein the cuvette (1) is made of plastic light-transmissive material.

6. The cuvette assembly of claim 1, wherein the cuvette (1) and/or the insert (5) is made of polystyrol, polyvinylchloride or polymethyl methacrylate.

7. The cuvette assembly of anyone of the preceding claims, wherein the number of projections (6) of the insert (5) corresponds to the number of wells (2) in the cuvette (1).

8. The cuvette assembly of anyone of the preceding claims, wherein the insert (5) when fitted into the cuvette (1) reduces the effective distance between the surface of the insert (5) and the inner surface of the cuvette (1) to 0,1 mm or less.

9. The cuvette assembly of anyone of the preceding claims, wherein the insert (5) carries on each projection (6) the same or a different activator, or
wherein not all projections (6) carry an activator.

10. The cuvette assembly of anyone of the preceding claims, wherein the insert (5) is made of plastic.

11. The cuvette assembly of anyone of the preceding claims, wherein the activator is a physiological activator or a non-physiological activator.

12. The cuvette assembly of claim 11, wherein said physiological activator is tissue plasminogen activator or urokinase plasminogen activator.

13. The cuvette assembly of claim 11, wherein said non-physiological activator is glass.

14. The cuvette assembly of anyone of the preceding claims, wherein the projections (6) are detachably or non-detachably connected to the area (7) of the insert (5).

15. The cuvette assembly of claim 1, wherein the cuvette (1) is wider than deep.

16. The cuvette assembly of claim 15, wherein the cuvette (1) has a width of approximately 1,1 mm.

17. Use of the cuvette assembly of anyone of claims 1 to 16 for monitoring spatial fibrin clot formation or dissolution *in vitro.*

18. The use of claim 17, which comprises the following method steps:
A) in case of clot formation
(a) Providing a cuvette containing one or more samples of blood plasma;
(b) Contacting plasma with a physiological activator of coagulation; and
(c) Register the growth of the fibrin clot in function of time and space.
B) in case of clot dissolution
(a) Providing a cuvette containing one or more samples of blood plasma comprising one or more fibrin clots;
(b) Contacting plasma with an activator of fibrinolysis; and
(c) Register the dissolution of the fibrin clot in function of time and space;
wherein either step (b) is-performed by inserting the insert (5) of the cuvette assembly into said cuvette (1).

19. The use of claim 18, wherein the method steps are performed at controlled temperature.

20. The use of claim 19, wherein the method steps are performed at 37°C.

21. The use of anyone of claims 18 to 20, wherein the activator is tissue factor, tissue plasminogen activator, thrombin, tissue factor expressing cells, tissue samples, glass or plastic.

## Patentansprüche

1. Eine Küvettenanordnung zur photometrischen Analyse, umfassend eine durch Trennwände (4) in eine Mehrzahl von Vertiefungen (2) unterteilte Küvette, charakterisiert durch einen Einsatz und einen Aktivator, wobei
der Einsatz (5) eine Vielzahl von Vorsprüngen (6) aufweist, welche aus einem Bereich (7) herausragen und welche an die Vertiefungen (2) der Küvette (1) angepasst sind; und
besagter Aktivator ausgewählt ist aus der Klasse der physiologischen Aktivatoren der Gerinnung, der nicht-physiologischen Aktivatoren der Gerinnung, der physiologischen Aktivatoren der Fibrinolyse und der nicht-physiologischen Aktivatoren der Fibrinolyse; und
wobei sich besagter Aktivator auf dem Einsatz der Vorsprünge (6) befindet.

2. Die Küvettenanordnung nach Anspruch 1, wobei sich besagter Aktivator an der unteren Kante (24) der Vorsprünge (6) befindet.

3. Die Küvettenanordnung nach Anspruch 1 oder 2, wobei die Vertiefungen (2) planparallele und / oder rechteckige Form in einer Querschnittsansicht zeigen.

4. Die Küvettenanordnung nach einem der vorhergehenden Ansprüche, wobei die Vertiefungen (2) der Küvette (1) in einer Linie angeordnet sind und gleiches Volumen aufweisen.

5. Die Küvettenanordnung nach einem der vorhergehenden Ansprüche, wobei die Küvette (1) aus einem lichtdurchlässigen Kunststoffmaterial besteht.

6. Die Küvettenanordnung nach Anspruch 1, wobei die Küvette (1) und / oder der Einsatz (5) aus Polystyrol, Polyvinylchlorid oder Polymethylmethacrylat besteht.

7. Die Küvettenanordnung nach einem der vorhergehenden Ansprüche, wobei die Anzahl der Vorsprünge (6) des Einsatzes (5) der Anzahl der Vertiefungen (2) in der Küvette (1) entspricht.

8. Die Küvettenanordnung nach einem der vorhergehenden Ansprüche, wobei der Einsatz (5), nach Einführen in die Küvette (1), den effektiven Abstand zwischen der Oberfläche des Einsatzes (5) und der Innenfläche der Küvette (1) auf 0,1 mm, oder weniger, verringert.

9. Die Küvettenanordnung nach einem der vorhergehenden Ansprüche, wobei der Einsatz (5) an jedem Vorsprung (6) den gleichen oder einen verschiedenen Aktivator trägt, oder wobei nicht alle Vorsprünge (6) einen Aktivator tragen.

10. Die Küvettenanordnung nach einem der vorhergehenden Ansprüche, wobei der Einsatz (5) aus Kunststoff ist.

11. Die Küvettenanordnung nach einem der vorhergehenden Ansprüche, wobei der Aktivator ein physiologischer Aktivator oder eine nicht - physiologischer Aktivator ist.

12. Die Küvettenanordnung nach Anspruch 11, wobei der physiologischer Aktivator Gewebe - Plasminogen - Aktivator oder Urokinase - Plasminogen - Aktivator ist.

13. Die Küvettenanordnung nach Anspruch 11, wobei der nicht - physiologischer Aktivator Glas ist.

14. Die Küvettenanordnung nach einem der vorhergehenden Ansprüche, wobei die Vorsprünge (6) lösbar oder unlösbar mit der Fläche (7) des Einsatzes (5) verbunden sind.

15. Die Küvettenanordnung nach einem der vorhergehenden Ansprüche, wobei die Küvette (1) breiter als tief ist.

16. Die Küvettenanordnung nach Anspruch 15, welche eine Breite von etwa 1.1 mm hat.

17. Verwendung einer Küvettenanordnung nach einem der Ansprüche 1 bis 16 zur Beobachtung der räumlichen Fibrin - Gerinnselbildung oder - Gerinselauflösung *in vitro.*

18. Die Verwendung gemäss Anspruch 17, welche die folgenden Verfahrensschritte umfasst:
(A) im Falle der Gerinnselbildung
(a) Bereitstellen einer Küvette die eine oder mehrere Proben von Blutplasma enthält;
(b) Kontaktieren des Plasmas mit einem physiologischen Aktivator der Gerinnung und
(c) Registrieren des Wachstums des Fibringerinnsels in Abhängigkeit von der Zeit und Raum;
(B) im Falle der Gerinnselauflösung
(a) Bereitstellen einer Küvette, die eine oder mehrere Proben von Blutplasma, umfassend eine oder mehrere Fibringerinnsel;
(b) Kontaktieren des Plasmas mit einem Aktivator der Fibrinolyse und
(c) Registrieren der Auflösung des Fibringerinnsels in Abhängigkeit von der Zeit und Raum;
wobei jeweils Schritt (b) durch das Einführen des Einsatzes (5) der Küvettenanordnung in besagte Küvette (1) durchgeführt wird.

19. Die Verwendung gemäss Anspruch 18, wobei die Verfahrensschritte bei kontrollierter Temperatur durchgeführt werden.

20. Die Verwendung gemäss Anspruch 19, wobei die Verfahrensschritte bei 37°C durchgeführt werden.

21. Die Verwendung gemäss einem der Ansprüche 18 bis 20, wobei der Aktivator Gewebefaktor, Gewebe - Plasminogen - Faktor, Thrombin, Gewebefaktor exprimierende Zellen, Gewebeproben, Glas oder Kunststoff ist.

## Revendications

1. Ensemble de cuve pour une analyse photométrique, comprenant une cuve segmentée en une multiplicité de puits (2) par des parois de segmentation (4), **caractérisé par** un insert et un activateur, dans lequel
ledit insert (5) comprend une multiplicité de saillies (6) protubérantes depuis une zone (7), qui sont adaptées pour s'ajuster dans les puits (2) de ladite cuve (1) ; et
ledit activateur est choisi dans la classe des activateurs physiologiques de coagulation, des activateurs non physiologiques de coagulation, des activateurs physiologiques de fibrinolyse ou des activateurs non physiologiques de fibrinolyse et dans lequel ledit activateur est situé sur les saillies d'insert (6).

2. Ensemble de cuve selon la revendication 1, dans lequel ledit activateur est situé sur le bord de dessous (24) des saillies (6).

3. Ensemble de cuve selon la revendication 1 ou 2, dans lequel les puits (2) sont parallèles au plan et/ou de forme rectangulaire dans une vue en coupe.

4. Ensemble de cuve selon l'une quelconque des revendications précédentes, dans lequel les puits (2) de la cuve (1) sont agencés en une ligne et ont un volume équitable.

5. Ensemble de cuve selon l'une quelconque des revendications précédentes, dans lequel la cuve (1) est constituée d'un matériau plastique transmettant la lumière.

6. Ensemble de cuve selon la revendication 1, dans lequel la cuve (1) et/ou l'insert (5) est constitué de polystyrol, de polychlorure de vinyle ou de polyméthacrylate de méthyle.

7. Ensemble de cuve selon l'une quelconque des revendications précédentes, dans lequel le nombre de saillies (6) de l'insert (5) correspond au nombre de puits (2) dans la cuve (1).

8. Ensemble de cuve selon l'une quelconque des revendications précédentes, dans lequel l'insert (5) lorsqu'il est ajusté dans la cuve (1) réduit la distance effective entre la surface de l'insert (5) et la surface interne de la cuve (1) à 0,1 mm ou moins.

9. Ensemble de cuve selon l'une quelconque des revendications précédentes, dans lequel l'insert (5) porte sur chaque saillie (6) un activateur identique ou différent, ou dans lequel les saillies (6) ne portent pas toutes un activateur.

10. Ensemble de cuve selon l'une quelconque des revendications précédentes, dans lequel l'insert (5) est constitué de plastique.

11. Ensemble de cuve selon l'une quelconque des revendications précédentes, dans lequel l'activateur est un activateur physiologique ou un activateur non physiologique.

12. Ensemble de cuve selon la revendication 11, dans lequel ledit activateur physiologique est un activateur tissulaire du plasminogène ou un activateur du plasminogène de type urokinase.

13. Ensemble de cuve selon la revendication 11, dans lequel ledit activateur non physiologique est le verre.

14. Ensemble de cuve selon l'une quelconque des revendications précédentes, dans lequel les saillies (6) sont raccordées de façon détachable ou non détachable à la zone (7) de l'insert.

15. Ensemble de cuve selon la revendication 1, dans lequel la cuve (1) est plus large que profonde.

16. Ensemble de cuve selon la revendication 15, dans lequel la cuve (1) a une largeur d'environ 1,1 mm.

17. Utilisation de l'ensemble de cuve de l'une quelconque des revendications 1 à 16 pour surveillance spatiale de la formation ou de la dissolution d'un caillot de fibrine in *vitro.*

18. Utilisation selon la revendication 17, qui comprend les étapes de procédé suivantes :
A) en cas de formation de caillot
(a) fourniture d'une cuve contenant un ou plusieurs échantillons de plasma sanguin ;
(b) mise en contact du plasma avec un activateur physiologique de coagulation ;
(c) enregistrement de la croissance du caillot de fibrine en fonction du temps et de l'espace ;
B) en cas de dissolution de caillot
(a) fourniture d'une cuve contenant un ou plusieurs échantillons de plasma sanguin comprenant un ou plusieurs caillots de fibrine ;
(b) mise en contact du plasma avec un activateur de fibrinolyse ; et
(c) enregistrement de la dissolution du caillot de fibrine en fonction du temps et de l'espace ;
dans laquelle l'une ou l'autre des étapes (b) est réalisée par insertion de l'insert (5) de l'ensemble de cuve dans ladite cuve (1).

19. Utilisation selon la revendication 18, dans laquelle les étapes de procédés sont réalisées à une température régulée.

20. Utilisation selon la revendication 18, dans laquelle les étapes de procédés sont réalisées à 37 °C.

21. Utilisation selon l'une quelconque des revendications 18 à 20, dans laquelle l'activateur est un facteur tissulaire, un activateur tissulaire du plasminogène, la thrombine, des cellules exprimant un facteur tissulaire, des échantillons de tissu, du verre ou du plastique.
